(19)

**Europäisches Patentamt**
**European Patent Office**
**Office européen des brevets**

(11) **EP 4 548 841 A1**

(12) **EUROPEAN PATENT APPLICATION**

(43) Date of publication:
**07.05.2025 Bulletin 2025/19**

(21) Application number: **23207896.4**

(22) Date of filing: **06.11.2023**

(51) International Patent Classification (IPC):
***A61B 5/024*** *(2006.01)*    ***A61B 5/349*** *(2021.01)*

(52) Cooperative Patent Classification (CPC):
**A61B 5/681; A61B 5/02416; A61B 5/349;**
**A61B 5/7267**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Kaunas University of Technology**
  **44249 Kaunas (LT)**
• **Vilnius University**
  **01513 Vilnius (LT)**

(72) Inventors:
• **Petrenas, Andrius**
  **Kaunas (LT)**
• **Sokas, Daivaras**
  **Kaunas (LT)**
• **Solosenko, Andrius**
  **Kaunas (LT)**
• **Butkuviene, Monika**
  **Kaunas (LT)**

• **Alves dos Santos Rodrigues, Ana Rita**
  **Kaunas (LT)**
• **Aidietis, Audrius**
  **Vilnius (LT)**
• **Bacevicius, Justinas**
  **Vilnius (LT)**
• **Juknevicius, Vytautas**
  **Vilnius (LT)**
• **Daukantas, Saulius**
  **Kaunas (LT)**
• **Marozas, Vaidotas**
  **Kaunas (LT)**
• **Abramikas, Zygimantas**
  **Vilnius (LT)**
• **Kersnauskaite, Dziugile**
  **Vilnius (LT)**
• **Janciuleviciute, Karolina**
  **Kaunas (LT)**

(74) Representative: **Pakeniene, Ausra**
  **AAA Law**
  **A. Gostauto street 40B**
  **03163 Vilnius (LT)**

(54) **A METHOD FOR IMPROVING ACCURACY AND RELIABILITY OF NON-MEDICAL WEARABLE DEVICES FOR GENERATING AN ALERT RELATED TO ACUTE MYOCARDIAL INFARCTION**

(57)    This disclosure presents a method for reducing the time required to initiate an alert on a suspected acute myocardial infarction (AMI) and improving the accuracy and reliability of the alert. The method involves acquiring and processing physiological signals, including multi-lead ECG and PPG signals captured using a wrist-worn device equipped with biooptical sensors and biopotential electrodes. These signals are utilized to extract both static and dynamic AMI features, such as ST segment abnormality index, QRS-T angle, the occurrence of bundle branch block, ECG signal quality, reduction in oxygen saturation, the number of ventricular premature beats, the occurrence of ventricular arrhythmias, and PPG signal quality index. These extracted AMI features are then employed as inputs to train a reservoir computing model, specifically an echo state neural network, using individualized data. The model computes an alert score, and if it surpasses a predefined threshold, an alert for suspected AMI is generated.

FIG. 1

EP 4 548 841 A1

**Description**

Technical field

The present invention relates to the field of generating an alert for suspected acute myocardial infarction using non-medical equipment, and more particularly, to a method for reducing the time required to initiate the alert and improving accuracy and reliability of an alert about a suspected acute myocardial infarction.

Background art

Acute myocardial infarction (AMI), also commonly known as a heart attack, is a serious health condition characterized by the sudden blockage of one or more coronary arteries that supply blood to the heart muscle. The blockage is typically caused by the formation of a blood clot, which impedes blood flow and causes damage to the heart muscle due to the lack of oxygen and nutrients. Symptoms of AMI can vary from person to person and may include chest pain, shortness of breath, nausea, vomiting, lightheadedness, and sweating. Prompt medical attention is crucial, as the condition can lead to severe complications or even death if left untreated. According to data from Eurostat (Eurostat. Avoidable Deaths in 2016 - For People Under 75, Two Deaths Out of Three in the EU Could Have Been Avoided. Eurostat News Release 136/2019), effective health management and timely interventions have the potential to prevent up to two-thirds of premature deaths, with AMI being the leading cause. Therefore, the development of new preventive strategies is a pressing priority.

Time is of the essence in the identification and treatment of AMI, as every minute that passes without intervention can result in further damage to the heart muscle. Nevertheless, some people are not aware of the symptom seriousness and are reluctant to seek medical examination. This is a worrying social issue that demands greater efforts to raise awareness about the life-threatening consequences of delayed treatment for those experiencing AMI-related symptoms.

AMI often affects the heart muscle and the heart's electrical conduction system, and these effects can be observed in an electrocardiogram (ECG). ECG clinical features characteristic of AMI depend on the location and extent of the heart muscle damage caused by the blockage of a coronary artery. Among the different waveforms and segments observed on an ECG, two that are altered by AMI are the QRS complex, representing ventricular depolarization, and the T wave, representing ventricular repolarization. Typically, AMI manifests as an elevation of the ST segment, which represents the period between ventricular depolarization and repolarization. ST elevation above the baseline is usually observed in the ECG leads corresponding to the location of the infarcted heart area. Hence, in clinical practice, when medical equipment is used and ECGs are interpreted by healthcare professionals, the primary focus is on analyzing changes in the ST segment of the ECG. In some cases, the ECG may show an inverted T wave in the leads affected by AMI. AMI can also lead to changes in the heart's electrical axis, causing shifts in the QRS complex and T-wave direction. These deviations can provide further clues about the extent and location of myocardial damage. AMI can disrupt the heart's electrical conduction system, leading to arrhythmias, such as frequent ventricular premature beats (VPBs), ventricular tachycardia, or ventricular fibrillation. Occasionally, AMI can lead to other conduction system-related issues, such as left or right bundle branch blocks.

A standard 12-lead ECG is the most readily accessible diagnostic test for detecting AMI. The 12-lead ECG is acquired using a set of 10 electrodes, with each lead corresponding to a different orientation of cardiac activity in three-dimensional space. Therefore, most infarctions originating in various regions of the heart can in principle be identified. The standard ECG leads are labeled as lead I, II, III, aVF, aVR, aVL, V1, V2, V3, V4, V5, and V6. Among these, leads I, II, III, aVR, aVL, and aVF are limb leads, whereas V1, V2, V3, V4, V5, and V6 are precordial leads. While a 12-lead ECG offers a comprehensive view of the heart's electrical activity, it still severely lacks reliability. For instance, the inter-rater agreement for diagnosing AMI from 12-lead ECG is only 0.33, indicating a lack of consensus among physicians. Diagnosing AMI with ST elevation, the sensitivity stands at 65%, while the specificity is 79% (McCabe, J.M., et al. "Physician Accuracy in Interpreting ST-Elevation Myocardial Infarction ECGs." J Am Heart Assoc 2.5 (2013): e000268). Another major limitation is the inability to obtain 12-lead ECG promptly in non-hospital settings following the onset of symptoms.

A reduced-lead ECG, such as a 6-lead ECG, may be used for prolonged (e.g., 1, or 2-day) ECG monitoring outside the clinical setting. Commonly, the 6-lead ECG is obtained from 6 electrodes including four limb and two chest electrodes. These leads capture the heart's electrical signals from various angles, although are less informative compared to the 12-lead ECG. While clinical reduced-lead ECG recorders may offer some benefits, they suffer from a limitation that the occurrence of an AMI cannot be predicted, making it difficult to prescribe ECG monitoring in advance. Therefore, there is a critical need for convenient and affordable technology that can facilitate the timely identification of AMI, and that is simple enough for people to use on their own.

Devices that rely on gathering fewer ECG leads than 12 leads have a fundamental limitation: certain infarctions may not be evident in the reduced-lead ECG. For instance, indications of an inferior wall AMI are frequently seen in limb leads II, III, and augmented limb lead aVF, while a posterior infarction may manifest in precordial leads V1 to V3, an anterior-septal infarction in precordial leads V1 to V4, and a lateral infarction in V5, V6, I, and aVL. Hence, a greater number of ECG leads is always preferred to avoid missing certain types of AMI.

Smartphones and smartwatches may be a convenient option since they are integral to most people's lives. Nevertheless, no breakthrough in AMI identification has yet been made despite the growing interest in applying smart devices for healthcare purposes. The latest smart devices are equipped with a feature to capture a single-lead ECG, but a single ECG lead alone does not provide the accuracy needed for AMI diagnosis, particularly when AMI occurs outside the inferior cardiac region. Accordingly, possibilities to obtain a 12-lead ECG using either portable accessory for smartphones, such as AliveCor (Muhlestein, J.B., et al. "Feasibility of Combining Serial Smartphone Single-Lead ECGs for ST-Elevation Myocardial Infarction Diagnosis." Am Heart J 221 (2020): 125-135.), or smartwatches with integrated ECG electrodes, such as Apple Watch Series 4 (Spaccarotella, C.A.M., et al. "Multichannel ECGs by Smartwatch for ST-Segment Change Diagnosis." JAMA Cardiol 5.10 (2020): 1176-1180) were studied. However, this principle suffers from a major drawback - single-lead ECGs must be recorded in succession rather than simultaneously to obtain a multi-lead ECG. Successive ECG acquisition is time-consuming and requires knowledge of correct device placement on the chest and abdomen, precluding proper signal acquisition during AMI when pain and anxiety are often experienced. Therefore, this core issue results in ECGs that lack synchronization and exhibit altered morphology due to inaccurate device placement, which, in turn, presents challenges in interpretation and poses the risk of diagnostic errors. Despite the resemblance of these modified ECG leads to the standard 12-lead ECG, they cannot, and should not, be employed for diagnostic purposes due to the absence of established guidelines for interpreting manually acquired modified leads. Moreover, the presence of motion artifacts and suboptimal skin-electrode contact further complicates the analysis of multi-lead ECG signals obtained through non-medical devices like smartwatches.

Current systems, methods, and apparatuses for detecting AMI using handheld devices have limitations. These limitations include being restricted to acquiring or analyzing a single or multiple lead ECG, as disclosed by patents US11419538B2, US11529085B1, US11213239B2, US8688189B2, and patent applications US2022015679A1, AU2016380869A1. Additionally, some systems, methods, and apparatuses involve analysis of blood oxygen saturation ($SpO_2$) data, as disclosed in patents US9402571B2, US9706952B2, and patent application US2008200774A1. The patent US9402571B2 also considers non-$SpO_2$ data, however, does not improve AMI detection. Patent applications with publication numbers WO2020/104986A2, EP3654347A1, and EP4105941 A1, disclose non-invasive systems and methods that include a wearable device with integrated biosensors capable of acquiring continuous photoplethysmogram (PPG) and intermitted ECG. These systems and methods do not offer means to improve the accuracy or reliability of AMI alerts.

It is crucial to consider that existing solutions for generating AMI alerts lack the integration of various physiological data, which could offer a more comprehensive assessment of AMI. Synergy becomes essential because different AMI features may not always be present in every case and often the opposite is true. For instance, ST elevation occurs in 30-40%, ST depression in 40-50%, increase in QRS-T angle in 10-20%, occurrence of left or right bundle branch block in 10-15%, drop in oxygen saturation in 10-15%, and occurrence of ventricular arrhythmias in 5-10% of patients with AMI. Therefore, relying on the AMI features collectively, rather than individually, is crucial for improving the accuracy and reliability of the automatically generated AMI alarm.

This invention overcomes specific limitations of the prior art, particularly in terms of reducing the time needed to acquire a multi-lead ECG and improving the accuracy and reliability of AMI-related alerts.

Brief description of the invention

The present invention introduces a method for reducing the time for obtaining multi-lead ECG and improving accuracy and reliability of alerts related to suspected AMI when non-medical wearable devices with fewer than 12 ECG leads are employed. Information missing due to reduced number of ECG leads, i.e., less than 12 leads, is compensated for by steps according to the invention.

The method does not encompass any diagnostic procedures and is not, in itself, a diagnostic method for AMI. The method involves analysis of physiological signals such as multi-lead ECG and PPGs of different wavelengths, for generating a prompt, accurate, and reliable alert for AMI.

The method steps that compensate for the missing information due to the reduced number of ECG leads are carried out on personal computing devices like personal computers, smartphones, or wrist-worn devices such as smartwatches, and a server. These steps comprises processing physiological signals, extracting static and dynamic AMI features, assessing ST segment abnormality and QRS-T angle in the ECG, detecting bundle branch block, calculating ECG signal quality index, estimating the reduction in oxygen saturation, detecting ventricular premature beats, detecting ventricular arrhythmia, calculating PPG signal quality index, training a reservoir computing model, such as an echo state neural network, on individual data. Various hyperparameters, such as reservoir size, spectral radius, sparsity, input scaling, output scaling, and noise level, are tuned to optimize the performance.

Subsequently, an alert on suspected AMI can be generated based on the data acquired when implementing method according to the invention if the alert score exceeds a predefined threshold. The alert may be conveyed using auditory, visual, or vibratory modalities. The generated alert, concerning a suspected AMI, complements the user's selfevaluation of own condition with objective information gathered by the non-medical device. In essence, the user receives an alert based on an analysis of a combination of diverse AMI-related features extracted from distinct physiological signals obtained by the non-medical device.

Brief description of the drawings

The unique and inventive aspects of this invention are detailed in the appended claims. The invention itself, however, may be best understood by referring to the following comprehensive description of the invention, which describes exemplary embodiments, given in non-restrictive examples, of the invention, taken in conjunction with the accompanying drawings, in which:

FIG. 1 shows a block diagram of method steps according to the invention.

FIG. 2 shows: a) an example of a wrist-worn device for use in the method according to the invention and b) a wrist-worn device-based 6-lead ECG and representative ECG beats obtained through weighted averaging.

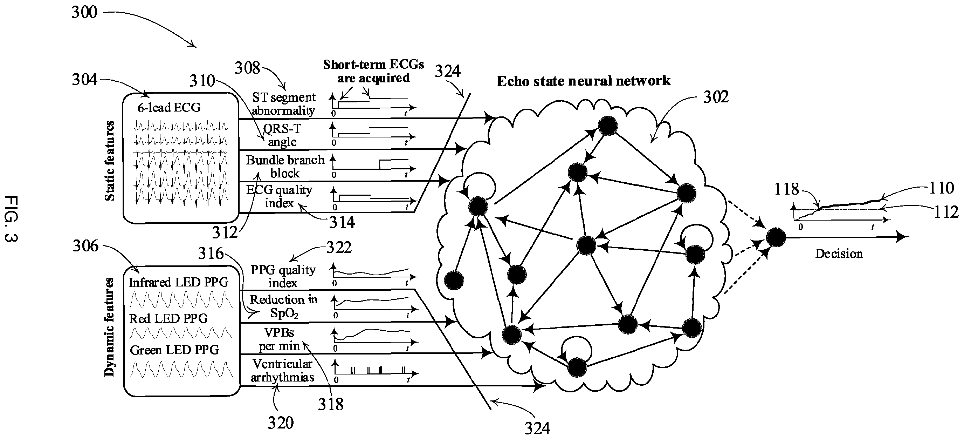

FIG. 3 shows an echo state network used in the method according to the invention.

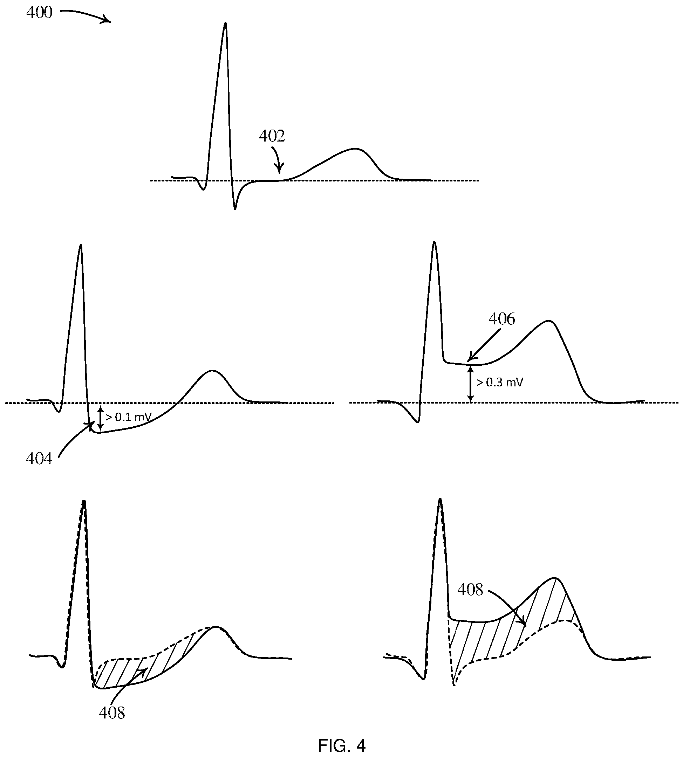

FIG. 4 shows the principle of assessing ST segment abnormality in the ECG.

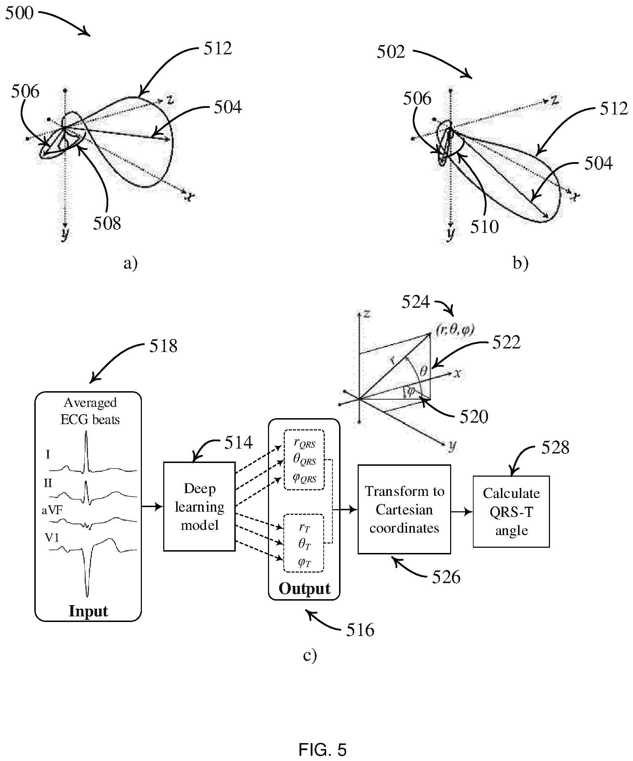

FIG. 5 shows the method steps for estimating the QRS-T angle in the ECG: a) QRS-T angle in AMI; b) normal QRS-T angle; c) application of deep learning model for estimating the QRS-T angle from a reduced-lead ECG.

FIG. 6 shows distinct features in the ECG associated with the left and right bundle branch block.

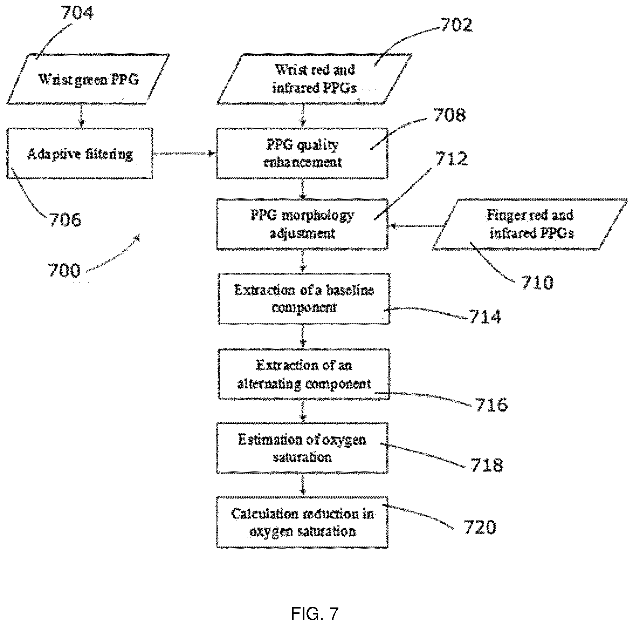

FIG. 7 shows the method steps for estimating the reduction in oxygen saturation in the PPG.

FIG. 8 shows: a) VPBs and ventricular arrhythmias in PPG; b) the method steps for detecting VPBs and ventricular arrhythmias.

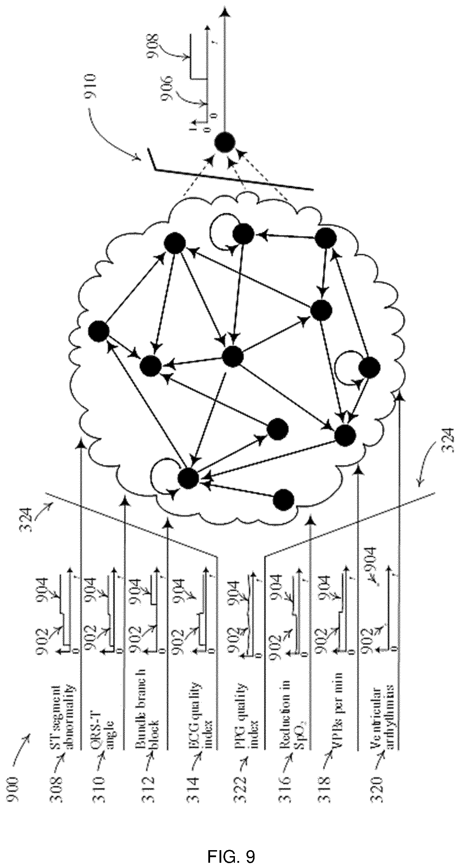

FIG. 9 shows the method steps for training an echo state network based on individual data.

Detailed description of the invention

The following terms are used to describe the embodiment of the invention:

- ECG refers to an electrocardiogram.
- PPG refers to a photoplethysmogram.
- LED refers to a light emitting diode.
- "Physiological signals" refers to multi-lead ECG and PPGs of various wavelengths.
- "AMI feature" refers to a specific measurable characteristic within a physiological signal that carries meaningful information about AMI. The AMI features are such as ST segment abnormality, an elevated QRS-T angle, the occurrence of new-onset bundle branch block, a decrease in $SpO_2$ levels, an increase in the frequency of ventricular premature beats per minute, or the occurrence of ventricular arrhythmias.
- "Static AMI features" refers to AMI features that are updated on-demand, e.g., only when the person actively chooses to acquire ECG. For example, the ST segment abnormality index retains its value until the individual deliberately initiates the acquisition of a multi-lead ECG. As soon as the multi-lead ECG is newly acquired, it undergoes analysis, and the ST segment abnormality index is updated.
- "Dynamic AMI features" refers to AMI features that are continuously updated without the active participation of the person. For example, the reduction in blood oxygen saturation levels is consistently updated with every heartbeat as long as the device remains on the wrist and continuously acquires PPG signals of green, red, and infrared wavelengths.

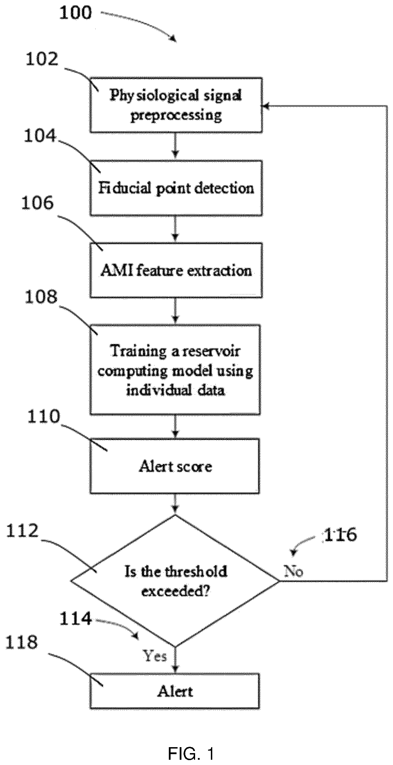

According to the embodiment of the invention and Fig. 1, steps of the method (100) for improving accuracy and reliability of non-medical wearable devices for generating an alert related to AMI are carried out on a personal computing device such as a standard personal computer, a portable computer, a handheld device, such as a smartphone, a wrist-worn device, such as a wristwatch, and a server.

The method (100) steps according to the invention comprise preprocessing (102) physiological signals obtained by a non-medical device, detecting fiducial points in the physiological signals (104), extracting AMI features (106), training a reservoir computing model using individual data (108).

An alert (118) on a suspected AMI can be produced (114) if an alert score (110), computed using data obtained while carrying out method steps according to the invention, exceeds a predefined threshold (112). Analysis steps are repeated if the threshold is not exceeded (116). An alert (118) may be implemented using audible, vibrating, visual, or a combination of multiple alert-producing modalities to promptly draw the attention of the person who uses the device.

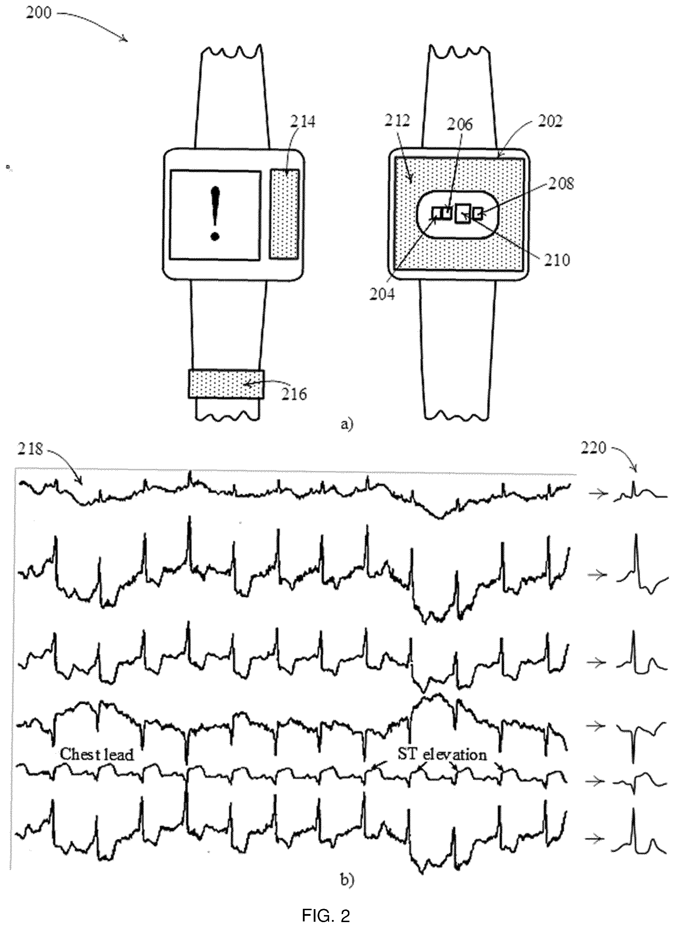

In the preferred embodiment of the invention and according to Fig.2, an example of a non-medical personal computing device is a wearable wrist-worn device (200) comprising integrated bio-optical sensors for capturing PPGs of different wavelengths and biopotential electrodes for acquiring multi-lead ECG signals. The bottom (202) of the wearable wrist-worn device (200) comprises a red-light LED (204), an infrared light LED (206), and a greenlight LED (208). Subsequently, a photodetector (210), disposed on the bottom (202) of the wearable wrist-worn device (200), captures the reflected lights to obtain PPG signals of corresponding wavelengths.

The incorporation of green light (approximately 525 nm wavelength) PPG, alongside the conventional red (approximately 660 nm wavelength) and infrared (approximately 940 nm wavelength) lights serves a dual purpose. Firstly, it enhances the quality of red light and infrared light PPG signals, particularly in settings susceptible to motion artifacts, low perfusion levels, or challenging environmental conditions, through the utilization of adaptive filtering techniques. Secondly, it enhances the robustness and reliability of PPG-based AMI feature extraction. Red, infrared, and green light PPG signals are preprocessed (102) using infinite impulse response filters. To remove high-frequency noise, a low-pass infinite impulse response filter with a cutoff frequency of 6 Hz is used. To remove baseline wandering, a high-pass infinite impulse response filter with a cutoff frequency of 0.5 Hz is applied. In the preferred embodiment, the embedded software within the wrist-worn device processes continuously acquired PPGs in real time.

A bottom biopotential electrode (212), located at the bottom of the wearable wrist-worn device (200), and a top biopotential electrode (214), located at the top of the wearable wrist-worn device (200), are configured to capture multi-lead ECG signals of which the top biopotential electrode (214) needs to be touched with a finger of the opposite hand. A further electrode (216), located at a distance from the top biopotential electrode (214), for example on a strap of the wearable wrist-worn device (200), must be brought into contact with chest of the wearer, specifically in the approximate location of precordial ECG lead V1. This arrangement allows the simultaneous acquisition of a 6-lead ECG of modified limb and chest leads (218).

In the preferred embodiment, the wrist-worn device is configured to acquire a raw multi-lead ECG signal (218), which is then transmitted to the server through an internet connection for preprocessing (102). During preprocessing (102), firstly, a finite impulse response filter configured with a cutoff frequency set at 40 Hz is used to attenuate high-frequency noise while preserving the frequency components inherent to the ECG signal. Secondly, an infinite impulse response filter configured with a cutoff frequency set at 0.05 Hz is used to mitigate ECG disturbances due to baseline wandering and artifacts resulting from suboptimal skin-electrode contact. Thirdly, the weighted averaging of ECG beats from multiple leads is used to obtain a representative ECG beat that is used for extracting static AMI features (220). Weighted averaging is accomplished by assigning varying weights to ECG beats derived from specific ECG leads, such as modified limb and chest leads, based on their noise level, expressed as the root mean square of signal values. ECG beats of superior quality with minimal noise are assigned higher weights, whereas those exhibiting a higher noise level are assigned lower weights. Signal to noise ratio is assessed in the TP segment in an ECG which represents the interval between the conclusion of the T wave and the onset of the P wave. Normally, the TP interval aligns with the baseline thus any deviations in the TP interval can be considered as being due to noise or artifacts.

In the preferred embodiment, the AMI alert (300) relies on a reservoir computing model such as an echo state neural network (302), capable of processing temporal data. The reservoir computing model utilizes static (304) and dynamic (306) AMI features as inputs. Static AMI features (304) may include ST segment abnormality index (308), QRS-T angle (310), the occurrence of bundle branch block (312), and ECG signal quality index (314), all of which are computed from the multi-lead ECG and updated with each intermittent ECG recording. Dynamic AMI features (306), such as reduction in oxygen saturation ($SpO_2$) (316), the number of ventricular premature beats per minute (318), the occurrence of ventricular arrhythmias (320), and PPG signal quality index (322), are updated continuously on a beat-to-beat basis. The numerical values of the ECG signal quality index (314) and the PPG signal quality index (322) are employed to adjust the importance of AMI features that are input into the reservoir computing model. In other words, when the signal quality index takes a low value, indicating poor signal quality, the importance of AMI features is proportionally reduced (324). The reservoir computing model produces an alert score (110), and when this score exceeds a predetermined threshold (112), it triggers the generation of an alert for a suspected AMI at that specific time instance (118).

Preprocessed (102) ECG and PPG signals are used to extract AMI features. In the preferred embodiment, the embedded software of the wrist-worn device (200) conducts real-time extraction of PPG-based AMI features, i.e., estimates a reduction in oxygen saturation ($SpO_2$) (316), the number of ventricular premature beats per minute (318), the occurrence of ventricular arrhythmias (320), and the quality of the PPG signal (322). These extracted PPG-based features, along with intermittent raw multi-lead ECG data, are transmitted to a secure server through an internet connection, facilitating further processing steps, including the extraction of ECG-based AMI features and the generation of an alert score. Detailed descriptions of the steps required to extract AMI features are provided below.

To extract distinctive AMI features from an ECG, the QRS-T complex has to be identified. To achieve this, several steps are taken. Firstly, the R peak within the QRS complex is pinpointed using the algorithm which encompasses bandpass filtering, differentiation, rectification, integration, and thresholding. Following the localization of the R peak, it serves as a reference point for determining both the termination of the T wave and the onset of the Q wave. A search for the T wave is conducted within a 200-millisecond window following the R peak. The method employed for detecting the T-wave endpoint employs a derivativebased approach. This technique involves the computation of the derivative of the ECG signal, with the endpoint of the T wave recognized as the point where the slope gradually recedes, signifying a return to the baseline. Similarly, the onset of the Q wave is identified using the same principle. By calculating the derivative of the ECG signal and identifying a notable shift in slope, the initiation of the Q wave is ascertained, indicating a distinct transition from the baseline.

The ST segment deviation (400) from the baseline of the ECG (402) is an important clinical feature of AMI. This deviation can manifest as either depression (404) or elevation (406), indicating abnormal electrical activity within the heart muscle, often associated with AMI. In clinical practice, ST depression is a more than 0.1 mV deviation of the ST segment below the baseline (404), whereas ST elevation is a deviation of more than 0.3 mV above the baseline (406). In the preferred embodiment of this invention, the method refrains from using predefined thresholds to identify ST segment abnormality since no step of diagnosis is involved. Instead, the numerical value of the ST abnormality will vary depending on the extent of the abnormality observed. This can be accomplished by calculating the abnormality index (308), which may involve determining the area (408) between the ST segment under analysis and a reference ST segment (402) acquired during a non-AMI condition. The resulting abnormality index provides a quantifiable measure of the observed abnormality, reflecting the degree of deviation from the baseline. To ensure robustness to noise, all acceptable-quality ST segments in the intermittent ECG are averaged and serve as a representative ST segment for subsequent calculations.

Alterations in the electrical properties of the affected region of the heart during an AMI can lead to changes in the QRS-T angle (500) of the ECG. The direction of the depolarization (QRS complex) and repolarization (T-wave) waves may deviate from their baseline spatial orientation (502), often increasing the angle between the QRS (504) and T (506) vectors, i.e., the spatial QRS-T angle (508). Normal QRS-T angles (510) typically range between 30-70$\underline{o}$, while angles exceeding 90$\underline{o}$ may suggest AMI.

The conventional approach for calculating the spatial QRS-T angle requires a vectorcardiogram to estimate the $xyz$ coordinates of the QRS ($\vec{u}_{QRS}$) and T ($\vec{u}_T$) vectors. The vectorcardiogram, composed by combining three orthogonal ECG leads, is a three-dimensional representation of the 12-lead ECG (512). These leads capture the electrical activity of the heart along the right-to-left (x), head-to-feet (y), and front-to-back (z) axes. At present, all existing methods for obtaining a vectorcardiogram necessitate the acquisition of two frontal and six precordial ECG leads to reconstruct the heart's electrical activity in three-dimensional space. The conventional approach for reconstructing the vectorcardiogram is not applicable when dealing with a modified 6-lead ECG obtained from a non-medical device, such as a wrist-worn device. Therefore, a deep learning model (514) is utilized to estimate $\vec{u}_{QRS}$ and $\vec{u}_T$. The model is a one-dimensional convolutional neural network with regression output trained with 12-lead ECGs to predict the three coordinates of the vectorcardiogram-derived QRS and T vectors. These QRS and T vectors serve as output (516) while predictions are generated from signal-averaged beat (518) of the subset of ECG leads acquired by the wrist-worn device serve as the input. The average beat serves as a representative QRS-T complex of all acceptable-quality beats in the intermittent ECG to ensure robustness to noise.

The deep-learning model is designed to determine the coordinates of $\vec{u}_{QRS}$ and $\vec{u}_T$, rather than directly calculating the angles. This approach is chosen to effectively utilize any spatial information present in the input ECG leads during the training process. To achieve this, a composite loss function is implemented to guide the model within the three-dimensional space. Since the direction of a vector in the $xyz$ space can be represented by two angles, azimuth $\varphi$ (520) and elevation $\theta$ (522), the model estimates $\vec{u}_{QRS}$ and $\vec{u}_T$ in spherical coordinates (524), i.e., $\vec{u}_{QRS} = (r_{QRS}, \theta_{QRS}, \varphi_{QRS})$ and $\vec{u}_T = (r_T, \theta_T, \varphi_T)$, using the composite loss function:

$$\mathcal{L} = w_1\left(\mathcal{L}_{\varphi_{QRS}} + \mathcal{L}_{\varphi_T}\right) + w_2\left(\mathcal{L}_{\theta_{QRS}} + \mathcal{L}_{\theta_T}\right) + w_3\left(\mathcal{L}_{r_{QRS}} + \mathcal{L}_{r_T}\right),$$

where $\mathcal{L}_{\varphi_{QRS}}$ and $\mathcal{L}_{\varphi_T}$ are the angular distance, $\mathcal{L}_{\theta_{QRS}}$ and $\mathcal{L}_{\theta_T}$ the cosine similarity between $\vec{u}_{QRS}$ and $\vec{u}_T$, and $\mathcal{L}_{r_{QRS}}$ and $\mathcal{L}_{r_T}$ are the distance between the projections of $\vec{u}_{QRS}$ and $\vec{u}_T$ in the xy plane. The hyperparamters $w_1$,

$w_2$ and $w_3$ weigh the penalization factor of $\mathcal{L}_\varphi, \mathcal{L}_\theta$ and $\mathcal{L}_r$. The coordinates of the estimated vectors are converted into Cartesian coordinates (526), i.e., $\vec{u}_{QRS} = (x_{QRS}, y_{QRS}, z_{QRS})$ and $\vec{u}_T = (x_T, y_T, z_T)$, to calculate the QRS-T angle (528).

Instead of relying on predetermined thresholds for defining abnormal QRS-T angles, the precise numerical value of the estimated QRS-T angle serves as an input to the reservoir computing model in the preferred embodiment of the invention.

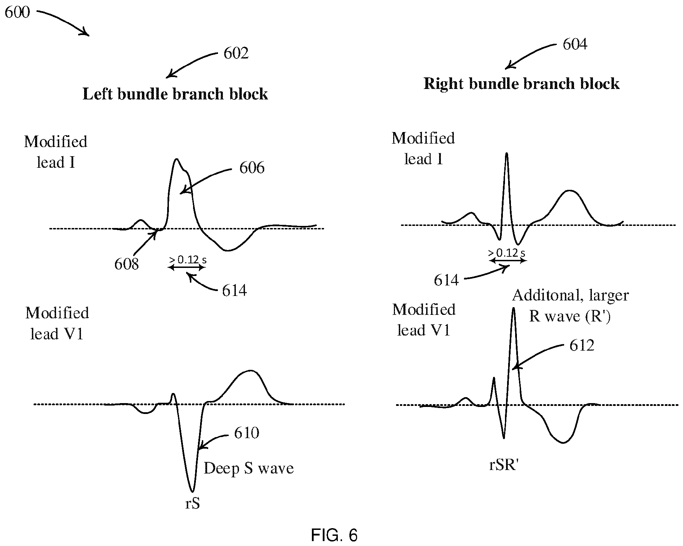

During AMI, the conduction system of the heart can be affected, leading to damage in the bundle branches (600), which are responsible for conducting electrical impulses through the ventricles of the heart. In the preferred embodiment of the invention and Fig. 6, a bundle branch block is detected in a multi-lead ECG acquired using a non-medical wrist-worn device (200), incorporating analysis of time and waveform features. The bundle branch block, as observed in the ECG, is characterized by a discernible ECG pattern in the modified leads I and V1 compared to the reference ECG obtained during a non-AMI condition. Particularly, the left bundle branch block (602) is distinguished by a broad monophasic R-wave in modified lead I (606), the absence of a Q-wave in modified lead I (608), and the presence of deep and broad S-waves in the modified lead V1 (610). The right bundle branch block (604) is distinguished by the presence of an rSR' complex, where the normal QRS complex is replaced by two R-waves and a large S-wave in the modified lead V1 (612). The initial R-wave is followed by a small S-wave, succeeded by a second prominent R-wave. Additionally, both left and right bundle branch blocks share a common feature: a prolonged QRS complex duration of equal to or greater than 0.12 seconds (614). The outcome of the bundle branch block detector is binary, providing a distinction between two possibilities: a value of "0" indicates the absence of a bundle branch block, while a value of "1" indicates the presence of a bundle branch block.

The quality assessment of the ECG signal involves calculating the ECG quality index, which compares how accurately different QRS detectors (e.g., Pan-Tompkins, Hamilton-Tompkins) detect QRS complexes. This is accomplished by using two or more QRS detectors on the ECG signal and checking if they detect the same QRS complex within the predefined error range (e.g., 10 ms). The quality index is calculated as follows:

$$\text{ECG quality index} = N_m/N_t,$$

where $N_m$ is the number of QRS complexes detected by the detectors within the predefined error range and $N_t$ is the total number of detected QRS complexes, obtained by summing the QRS complexes detected by all the QRS detectors without counting duplicate matches. In the preferred embodiment, the analysis window size is set to match the duration of the entire ECG signal. ECG quality is assessed based on the specific numerical value of the ECG quality index, rather than using predefined thresholds. The ECG quality index is represented by a numerical value ranging from 0 to 1. A value of 0 indicates poor quality, while a value approaching 1 signifies good quality. This numerical value is then used to adjust the significance of other ECG-based AMI features when they are input into the reservoir computing model (324). In this context, each ECG-based input (such as the ST segment abnormality index, QRS-T angle, or the presence of bundle branch block) is scaled by the ECG quality index value. When the ECG quality is poor, e.g., the value of the ECG quality index approaches 0, these inputs become significantly less influential and contribute negligibly to the AMI alert score.

In the preferred embodiment, unlike the typical method of estimating oxygen saturation ($SpO_2$) using finger PPG signals, the reduction in $SpO_2$ is estimated from wrist PPG signals. The principle of $SpO_2$ estimation is rooted in the fact that hemoglobin absorbs red and infrared light differently depending on whether it is bound to oxygen or not. Oxygenated hemoglobin absorbs more infrared wavelength and less red wavelength, while deoxygenated hemoglobin absorbs more red wavelength and less infrared wavelength.

Estimation of $SpO_2$ (700) from the wrist PPGs of red and infrared wavelength is challenging due to low blood perfusion and mixing of arterial and venous blood in the light path. Hence, two steps are undertaken to enhance the information content of the wrist PPG signals (702). Firstly, a green wavelength PPG (704), which is often of better quality, serves as a reference for a normalized least-mean-square adaptive filter (706) of first order with the adaptation step of 0.1 to improve the quality of red and infrared PPG signals (708). Secondly, the morphology of the wrist PPG signal (712) is modified to align with the morphology of the finger PPG signal. To achieve this, a system identification method, such as the autoregressive moving average (ARMA) model, is employed to establish the relationship between finger and wrist PPG signals. The ARMA model estimates the individual transfer function by utilizing wrist PPG signals as the input and finger PPG signals (710) as the output of the system. Subsequently, the estimated transfer function, essentially a frequency response function, is applied to independently process wrist PPG signals, eliminating the need for acquiring finger PPG data through conventional inconvenient means.

In the next step, red and infrared wavelength PPG signals are processed to extract the alternating component (AC) from the baseline component (DC). The baseline component represents the steady-state blood volume level in the tissue, and it is a result of the average blood volume in the microvascular bed. The baseline component is relatively constant during the cardiac cycle and exhibits only slow-changing oscillations. To extract the baseline component (714), the first step involves removing high-frequency noise using a low-pass filter with a cutoff frequency of 6 Hz. Next, a normalized least-mean-square adaptive filter of a first order with an adaptation step of 0.01 is applied. The alternating component represents the pulsatile changes in blood volume associated with each heartbeat. To extract the alternating component (716), a high-pass filter with a cutoff frequency of 0.5 Hz and a normalized least-mean-square adaptive filter of a first-order with an adaptation step of 0.1 are used. The ratio of the amplitudes of the alternating and baseline components of the red and infrared PPG signals is proportional to the amount of oxygenated hemoglobin in the blood. Oxygen saturation (718) is estimated using an empirical calibration curve based on measurements obtained from a population of individuals with known oxygen saturation levels and is found by

$$SpO_2 = 115 - 30 \times \frac{\text{rms}\,(AC_{\text{red}})}{\text{rms}\,(DC_{\text{red}})} \times \frac{\text{rms}\,(DC_{\text{infrared}})}{\text{rms}\,(AC_{\text{infrared}})}.$$

Reduction in SpO$_2$ (720) is calculated as follows:

$$rSpO_2 = nSpO_2 - SpO_2,$$

where $nSpO_2$ represents the oxygen saturation value under normal conditions, such as those obtained during a calibration measurement during a non-AMI condition.

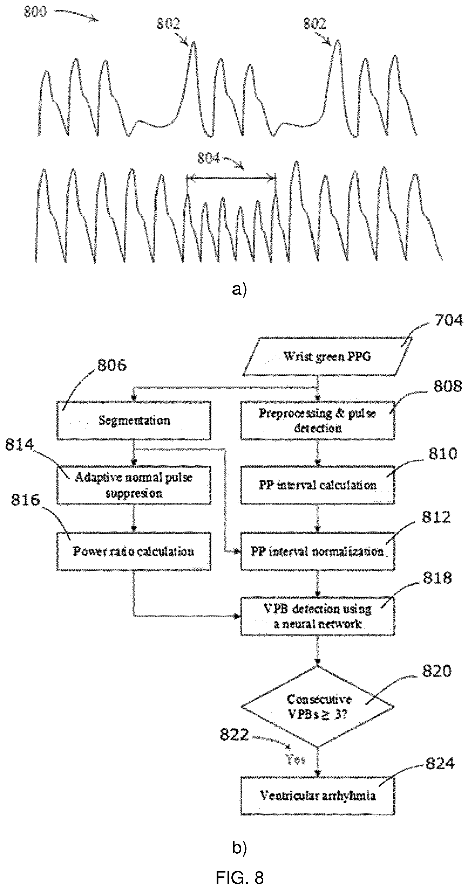

During an AMI, the damage in the heart muscle may lead to the development of various heart rhythm disturbances (800) such as frequent VPBs (802) and ventricular arrhythmias (804) such as ventricular tachycardia, ventricular flutter, and ventricular fibrillation. In the preferred embodiment, ventricular arrhythmias, including VPBs, are detected based on certain characteristics observed on a PPG signal recorded at a green wavelength. According to the embodiment of the invention and Fig. 8, the algorithm for detecting VPBs utilizes temporal and power-derived features extracted from an individual PPG pulse of green light PPG (704). A sliding analysis window of 10 s with 50% overlap is used for PPG segmentation (806). Positive peaks of the preprocessed PPG are detected using a threshold-crossing technique (808). The intervals between PPG pulses, i.e., PP intervals (810), are normalized (812) to make them independent of heart rate, and a normalization principle based on the ratio of the current and mean values of the intervals is employed. A second high-pass finite impulse response filter with a variable cutoff frequency is used to extract higher frequency components, and a pre-whitened least squares adaptive filter is employed with an order of 12 and a forgetting factor of 0.999 to suppress normal PPG pulses (814) and in such a way to enhance premature pulses which are likely VPBs. Power ratios (816) are computed in segments between adjacent PPG pulses, involving both preprocessed PPG and the signal after adaptive filtering. VPBs are detected using a feed-forward artificial neural network with a hidden layer of 50 neurons, trained using a backpropagation learning technique (818).

Ventricular arrhythmias, such as ventricular tachycardia and ventricular flutter, are characterized by an accelerated heart rate (804) resulting from irregular electrical impulses arising from the ventricles of the heart. Ventricular tachycardia is typically accompanied by a regular heart rhythm that exceeds 100 beats per minute, while ventricular flutter also exhibits a regular rhythm with a range commonly falling between 250 and 350 beats per minute. If three or more consecutive VPBs in the PPG (820) with a heart rhythm exceeding 100 beats per minute are detected (822), the entire episode of VPBs is assigned to ventricular arrhythmia (824), and it results in a high output ("1") being generated for this AMI feature. Otherwise, a low output ("0") is generated.

The quality of PPG signals can be affected by various factors, including motion artifacts, ambient light, skin pigmentation, and sensor placement. In the preferred embodiment, PPG pulse quality is assessed using template matching involving comparing the shape of a reference pulse waveform with the shape of the measured pulse waveform. Obtaining a reference pulse waveform may be done by averaging several high-quality PPG pulse waveforms. The reference waveform should represent the typical shape of a high-quality PPG pulse. Preprocessing of the measured pulse waveform involves filtering to remove noise, baseline correction, and normalization to ensure that the waveform has a consistent amplitude and duration. Aligning the reference waveform with the measured waveform can be done using cross-correlation or other alignment methods to ensure that the reference waveform is properly aligned with the corresponding portion of the measured waveform. Calculating the similarity score, which represents how closely the measured waveform matches the reference waveform, can be done using a similarity metric such as the correlation coefficient. This numerical value is subsequently employed to modify the relative importance of various PPG-based AMI features when they are fed into the reservoir computing model (324). In this context, each PPG-based input (e.g., reduction in SpO$_2$, the count of ventricular premature beats per minute, and the occurrence of ventricular arrhythmias) is proportionally weighted by the PPG quality index value. When the PPG quality is low, for instance, as the PPG quality index value approaches 0, these inputs exert substantially less influence and make only a negligible contribution to the AMI alert score.

The echo state neural network is utilized as the decision-making model in this embodiment of the invention based on its distinct advantages over conventional machine learning and deep learning approaches. The echo state neural network offers ease of training, as it employs fixed, randomly initialized recurrent weights and only output weights adjusted during training. The echo state neural network is computationally efficient, e.g., can be parallelized for efficient implementation on hardware accelerators, making it suitable for applications with limited computational resources, such as wrist-worn devices. The echo state neural network exhibits robustness to noise and can effectively work with noisy data, while also demonstrating good generalization to unseen data. The performance of an echo state network depends on several hyperparameters that need to be tuned to achieve optimal performance.

In a preferred embodiment, the size of the reservoir, which is the recurrent layer of the echo state network, is set to 1000. The spectral radius, which is a scaling factor that is applied to the weights of the reservoir and affects the stability and dynamic range of the network, is set to 1. The sparsity of the reservoir, which is the proportion of zero-valued weights in the reservoir, is set to 20 %. Input scaling, which is a scaling factor applied to the input weights to affect the sensitivity of the network to the input data, is set to 1. The output scaling, which refers to the transformation applied to the activations of the neurons in the reservoir to produce the final network output, is set to 1. The noise level, which may improve the robustness of the network and prevent overfitting, is set to 0.001.

The goal of training (900) the echo state network is to enable the model to recognize changes in AMI features compared to those estimated during a non-AMI condition. The training process may involve a sequence of 60 non-pathological cardiac cycles (902) for each person's data which may be obtained before the occurrence of AMI. Then, a deviation in AMI features is added for another 60 cardiac cycles to mimic AMI (904). Specifically, the ST segment abnormality index (308) is increased by 50%, the QRS-T angle (310) is increased by 50%, the occurrence of bundle branch block (312) is identified, i.e., the output of this particular feature is set to "1", $SpO_2$ is reduced by 10 % (316), the number of VPBs per min (318) is increased by 20% compared to baseline numbers of a particular person, and the occurrence of ventricular arrhythmias (320) is identified, i.e., the output of this particular feature is set to "1". The values of ECG (314) and PPG (322) signal quality indexes from non-pathological cardiac cycles are repeated for artificially constructed pathological cardiac cycles. The training data for the echo state network consists of input AMI features and a target output signal that is constructed. In the target output signal, a low level ("0") is assigned to non-AMI features (906), while a high level ("1") is assigned to artificially constructed AMI features that reflect extreme deviation (908). The output weights may be trained using various techniques, such as Ridge regression or pseudoinverse (910).

When a new set of features is provided as input to the echo state network, it undergoes a forward pass through the dynamic reservoir of neurons and output layers. The network evaluates the input AMI features and produces output values that indicate the likelihood of an AMI. A high output value, approaching 1, signifies a high probability of AMI. To warn the user, an alert is triggered when the network output surpasses the predefined threshold value of 0.5.

In the disclosed invention, the interaction between the server and the wrist-worn device is facilitated through a bidirectional communication channel. The wrist-worn device transmits physiological data, including ECG signals and dynamic AMI features extracted from the wrist PPGs to the secure server via an internet connection established either through a smartphone or a GSM module integrated into the wrist-worn device. Upon receiving and processing this data, the server autonomously extracts static AMI features from the ECG signals. Subsequently, the server provides real-time feedback to the wrist-worn device, promptly issuing a notification alert to the device on a suspected AMI.

The preferred embodiment has been described above however it is important to note that modifications can be made to these embodiments without deviating from the scope of the invention as defined by the claims. For instance, the sequence in which method steps are performed can be altered in alternative embodiments, and certain method steps may be omitted entirely in other alternative embodiments. Optional features mentioned in device and system embodiments may be present in some embodiments while absent in others. Thus, the preceding description is primarily provided for illustrative purposes and should not be construed as limiting the scope of the invention as defined in the claims.

**Claims**

1. A method for improving the accuracy and reliability of non-medical wearable computing devices for generating an alert related to acute myocardial infarction comprises acquiring continuous photoplethysmogram (PPG) of green light, red light, and infrared light by a photodetector and intermittent multi-lead electrocardiogram (ECG) from electrodes by the non-medical wearable computing device and processing the so acquired physiological signal data in a server for generating an alert related to acute myocardial infarction **characterized in that** the method further comprises

   - preprocessing ECG signals, where
   weighted averaging of ECG beats (220) from multiple leads is used to attenuate high-frequency noise and mitigate ECG disturbances due to baseline wandering and artifacts resulting from suboptimal skin-electrode contact to obtain a representative ECG beat of improved quality;
   - enhancing the estimation of the reduction in $SpO_2$ by

      providing green wavelength PPG (704) as a reference for a normalized least-mean-square adaptive filter (706) of first order with the adaptation step of 0.1 to improve the quality of red and infrared PPG signals (708), modifying morphology of the wrist PPG signal (712) by applying an autoregressive moving average model to establish the relationship between finger and wrist PPG signals by estimating individual transfer function by utilizing wrist PPG signals as the input and finger PPG signals (710) as the output of the model, and using an estimated frequency response function to independently process wrist PPG signals, eliminating the need for acquiring finger PPG signals;

- improving the separation of the alternating component from the baseline component in processed red and infrared wavelength PPG signals by applying a first-order normalized least-mean-square adaptive filter with an adaptation step of 0.01;
- assessing PPG pulse quality by calculating the PPG quality index by obtaining a reference pulse waveform by averaging high-quality PPG pulse waveforms and aligning the reference waveform with the measured waveform, where the PPG quality index is represented by a numerical value ranging from 0 to 1 and used to adjust the significance of other PPG-based AMI features when they are input into the reservoir computing model for scaling each PPG-based input by the PPG quality index value;

- assessing ECG signal quality by calculating the ECG quality index which compares the accuracy of detected QRS complexes by different QRS detectors, where the ECG quality index is represented by a numerical value ranging from 0 to 1 and used to adjust the significance of other ECG-based AMI features when they are input into the reservoir computing model for scaling each ECG-based input by the ECG quality index value;

- training an echo state neural network (302) using static (304) and dynamic (306) AMI features as inputs

where static AMI features (304) such as ST segment abnormality index (308), QRS-Tangle (310), occurrence of bundle branch block (312), and ECG signal quality index (314), are computed from the multi-lead ECG and updated with each ECG recording,

where dynamic AMI features (306), such as reduction in oxygen saturation (SpO2) (316), the number of ventricular premature beats per minute (318), the occurrence of ventricular arrhythmias (320), and PPG signal quality index (322), are updated continuously on a beat-to-beat basis.

2. Method according to claim 1, where weighted averaging of ECG beats from multiple leads comprises assigning varying weights to ECG beats derived from modified chest and limb ECG leads based on their noise level, expressed as the root mean square of signal values, where ECG beats of superior quality with minimal noise are assigned higher weights, whereas those exhibiting a higher noise level are assigned lower weights, and signal to noise ratio is assessed in the TP segment in an ECG which represents the interval between the conclusion of the T wave and the onset of the P wave.

3. Method according to any one of the preceding claims, where ST segment abnormality index (308) is calculated by determining the area (408) between the ST segment under analysis and a reference ST segment (402) acquired during a non-AMI condition, where all acceptable-quality ST segments in the intermittent ECG are averaged and used as a representative ST segment for subsequent calculations.

4. Method according to any one of the preceding claims, where calculating the spatial QRS-Tangle comprises estimating the $xyz$ coordinates of the QRS ($\vec{u}_{QRS}$) and T ($\vec{u}_T$) vectors, where the deep learning model (514) is utilized to estimate $\vec{u}_{QRS}$ and $\vec{u}_T$ where the model is a one-dimensional convolutional neural network with regression output trained with 12-lead ECGs to predict the three coordinates of the vectorcardiogram-derived QRS and T vectors, where QRS and T vectors serve as output (516) while predictions are generated from signal-averaged beat (518) of the subset of ECG leads acquired by the wrist-worn device serve as the input, where the average beat serves as a representative QRS-T complex of all acceptable-quality beats in the intermittent ECG to ensure robustness to noise, where the deep-learning model determines the coordinates of $\vec{u}_{QRS}$ and $\vec{u}_T$ using a composite loss function for guiding the model within the three-dimensional space, where the direction of a vector in the $xyz$ space is represented by azimuth $\varphi$ (520) and elevation $\theta$ (522), the model estimates $\vec{u}_{QRS}$ and $\vec{u}_T$ in spherical coordinates (524) using the composite loss function:

$$\mathcal{L} = w_1\left(\mathcal{L}_{\varphi_{QRS}} + \mathcal{L}_{\varphi_T}\right) + w_2\left(\mathcal{L}_{\theta_{QRS}} + \mathcal{L}_{\theta_T}\right) + w_3\left(\mathcal{L}_{r_{QRS}} + \mathcal{L}_{r_T}\right),$$

where $\mathcal{L}_{\varphi_{QRS}}$ and $\mathcal{L}_{\varphi_{T}}$ are the angular distance, $\mathcal{L}_{\theta_{QRS}}$ and $\mathcal{L}_{\theta_{T}}$ the cosine similarity between $\vec{u}_{QRS}$ and $\vec{u}_{T}$, and $\mathcal{L}_{r_{QRS}}$ and $\mathcal{L}_{r_{T}}$ are the distance between the projections of $\vec{u}_{QRS}$ and $\vec{u}_{T}$ in the *xy* plane, where the hyperparameters $w_1$, $w_2$ and $w_3$ weigh the penalization factor of $\mathcal{L}_{\varphi}, \mathcal{L}_{\theta}$ and $\mathcal{L}_{r}$, and the coordinates of the estimated vectors are converted into Cartesian coordinates (526) for calculating the QRS-T angle (528).

5. Method according to any one of the preceding claims, where the ECG quality index is calculated by

$$\text{ECG quality index} = N_m/N_t,$$

where $N_m$ is the number of QRS complexes detected by the detectors within the predefined error range and $N_t$ is the total number of detected QRS complexes, obtained by summing the QRS complexes detected by all the QRS detectors without counting duplicate matches, where the analysis window size is set to match the duration of the entire intermittent ECG signal.

6. A method according to any one of the preceding claims, where a bundle branch block is detected by analyzing the ECG signal morphology of modified I and V1 leads for providing a binary outcome of "0" for the absence of a bundle branch block or "1" for its presence.

7. Method according to any one of the preceding claims, where ventricular premature beats (VPBs) are detected in the PPG signal of a green wavelength by:

   - segmenting the PPG signal into individual PPG pulses and detecting positive peaks within said individual PPG pulses using a threshold-crossing technique;
   - normalizing the intervals between said individual PPG pulses to make them independent of heart rate, employing a normalization principle based on the ratio of the current and mean values of the intervals;
   - utilizing a pre-whitened least squares adaptive filter with an order of 12 and a forgetting factor of 0.999 to suppress normal PPG pulses and enhance VPBs;
   - computing power ratios in segments between adjacent PPG pulses, involving both preprocessed PPG and the signal after adaptive filtering;
   - training a feed-forward artificial neural network with a hidden layer of 50 neurons;
   - determining the presence of ventricular arrhythmias, including ventricular tachycardia and ventricular flutter, by identifying three or more consecutive VPBs with a heart rhythm exceeding 100 beats per minute within the PPG signal;
   - generating a binary value of "1" when ventricular arrhythmia is detected, and a binary value of "0" when ventricular arrhythmia is not detected.

8. Method according to any one of the previous claims, where training the echo state neural network (302) using static (304) and dynamic (306) AMI features as inputs comprise:

   - obtaining a sequence of 60 non-pathological cardiac cycles (902) for each person's data before the occurrence of AMI, and
   - adding deviation in AMI features for another 60 cardiac cycles to mimic AMI (904), comprising increasing the ST segment abnormality index (308) by 50%, increasing the QRS-T angle (310) by 50%, setting the occurrence of bundle branch block to "1" (312), reducing $SpO_2$ by 10 % (316), increasing the number of VPBs per min (318) by 20% compared to baseline numbers of a particular person, and setting the occurrence of ventricular arrhythmias to "1" (320).

9. Method according to any one of the previous claims, where size of a recurrent layer of the echo state network is set to 1000, a scaling factor that is applied to the weights of the reservoir is set to 1, the sparsity of the reservoir, which is the proportion of zero-valued weights in the reservoir, is set to 20 %, a scaling factor applied to the input weights is set to 1, the transformation applied to the activations of the neurons in the reservoir to produce the final network output is set to 1, and the noise level is set to 0.001.

FIG. 1

FIG. 2

FIG. 3

FIG. 4

a)

b)

c)

FIG. 5

FIG. 6

704

Wrist green PPG

702

Wrist red and
infrared PPGs

708

Adaptive filtering → PPG quality
enhancement

706

712

PPG morphology
adjustment ← Finger red and
infrared PPGs

700

710

Extraction of a baseline
component

714

Extraction of an
alternating component

716

Estimation of oxygen
saturation

718

Calculation reduction in
oxygen saturation

720

FIG. 7

a)

b)

FIG. 8

FIG. 9

Europäisches
Patentamt

European
Patent Office

Office européen
des brevets

# EUROPEAN SEARCH REPORT

**Application Number**

EP 23 20 7896

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/100693 A1 (VELO LINO [US]) 2 April 2020 (2020-04-02) * paragraphs [0026] - [0063], [0077] - [0107]; claims 1-3; figures 1-2,5-6 * | 1 | INV. A61B5/024 A61B5/349 |
| X | US 2023/079489 A1 (CHO SUNGHWAN [KR] ET AL) 16 March 2023 (2023-03-16) * the whole document * | 1 | |
| X | WO 2023/018318 A1 (HEXACHECK INC [KR]) 16 February 2023 (2023-02-16) * the whole document * | 1 | |
| X | US 2021/137392 A1 (HWANG IN-DUK [KR]) 13 May 2021 (2021-05-13) * the whole document * | 1 | |
| X | US 2023/009430 A1 (STEPHENS PHILIP [US]) 12 January 2023 (2023-01-12) * the whole document * | 1 | |
| A | US 2022/287613 A1 (ALBERT DAVID E [US]) 15 September 2022 (2022-09-15) * paragraphs [0102] - [0107] * | 4 | **TECHNICAL FIELDS SEARCHED (IPC)**<br>A61B |
| A | FRANCO GIUSEPPE ET AL: "Continuous Blood Pressure Estimation Through Optimized Echo State Networks", 9 September 2019 (2019-09-09), ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 48 - 61, XP047520740, ISBN: 978-3-319-10403-4 [retrieved on 2019-09-09] * the whole document * | 9 | |

The present search report has been drawn up for all claims

| Place of search | Date of completion of the search | Examiner |
|---|---|---|
| Berlin | 4 April 2024 | Kronberger, Raphael |

**EP 4 548 841 A1**

## ANNEX TO THE EUROPEAN SEARCH REPORT
## ON EUROPEAN PATENT APPLICATION NO.

EP 23 20 7896

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2024

| Patent document cited in search report | | | Publication date | Patent family member(s) | | Publication date |
|---|---|---|---|---|---|---|
| US 2020100693 | A1 | | 02-04-2020 | NONE | | |
| US 2023079489 | A1 | | 16-03-2023 | KR | 20220053364 A | 29-04-2022 |
| | | | | US | 2023079489 A1 | 16-03-2023 |
| | | | | WO | 2022086273 A1 | 28-04-2022 |
| WO 2023018318 | A1 | | 16-02-2023 | KR | 20230025371 A | 21-02-2023 |
| | | | | WO | 2023018318 A1 | 16-02-2023 |
| US 2021137392 | A1 | | 13-05-2021 | GB | 2589990 A | 16-06-2021 |
| | | | | GB | 2614462 A | 05-07-2023 |
| | | | | JP | 7277970 B2 | 19-05-2023 |
| | | | | JP | 2021530276 A | 11-11-2021 |
| | | | | JP | 2023099105 A | 11-07-2023 |
| | | | | KR | 20200001823 A | 07-01-2020 |
| | | | | US | 2021137392 A1 | 13-05-2021 |
| | | | | WO | 2020005027 A1 | 02-01-2020 |
| US 2023009430 | A1 | | 12-01-2023 | US | 2021052229 A1 | 25-02-2021 |
| | | | | US | 2023009430 A1 | 12-01-2023 |
| | | | | WO | 2021034467 A1 | 25-02-2021 |
| US 2022287613 | A1 | | 15-09-2022 | CN | 113056231 A | 29-06-2021 |
| | | | | EP | 3852621 A1 | 28-07-2021 |
| | | | | JP | 2021535810 A | 23-12-2021 |
| | | | | US | 2021267525 A1 | 02-09-2021 |
| | | | | US | 2022287613 A1 | 15-09-2022 |
| | | | | WO | 2020060780 A1 | 26-03-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11419538 B2
- US 11529085 B1
- US 11213239 B2
- US 8688189 B2
- US 2022015679 A1
- AU 2016380869 A1
- US 9402571 B2
- US 9706952 B2
- US 2008200774 A1
- WO 2020104986 A2
- EP 3654347 A1
- EP 4105941 A1

**Non-patent literature cited in the description**

- **MCCABE, J.M. et al.** Physician Accuracy in Interpreting ST-Elevation Myocardial Infarction ECGs.. *J Am Heart Assoc*, 2013, vol. 2 (5), e000268
- **MUHLESTEIN, J.B. et al.** Feasibility of Combining Serial Smartphone Single-Lead ECGs for ST-Elevation Myocardial Infarction Diagnosis. *Am Heart J*, 2020, vol. 221, 125-135
- **SPACCAROTELLA, C.A.M. et al.** Multichannel ECGs by Smartwatch for ST-Segment Change Diagnosis. *JAMA Cardiol*, 2020, vol. 5 (10), 1176-1180